(19) Europäisches Patentamt
European Patent Office
Office européen des brevets

(11) **EP 2 525 207 B1**

(12) **EUROPÄISCHE PATENTSCHRIFT**

(45) Veröffentlichungstag und Bekanntmachung des Hinweises auf die Patenterteilung:
**19.06.2019   Patentblatt 2019/25**

(51) Int Cl.:
***G01N 1/12*** *(2006.01)*      ***G01N 33/20*** *(2019.01)*

(21) Anmeldenummer: **12002651.3**

(22) Anmeldetag: **17.04.2012**

(54) **Probennehmer für die Probennahmen aus Schmelzen mit einem Schmelzpunkt größer 600°C sowie Verfahren zur Probennahme**

Sampler for taking samples of melts with a melt point higher than 600°C and sampling method

Échantillonneur pour le prélèvement d'échantillons issus d'une fusion avec un point de fusion supérieur à 600 °C et procédé de prélèvement d'échantillons

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: 16.12.2011   DE 102011121183
18.05.2011   DE 102011101943

(43) Veröffentlichungstag der Anmeldung:
**21.11.2012   Patentblatt 2012/47**

(73) Patentinhaber: **Heraeus Electro-Nite International N.V.**
**3530 Houthalen (BE)**

(72) Erfinder:
• **Song, Lihuan**
**3920 Lommel (BE)**
• **Broekmans, Gerrit**
**3583 Paal (BE)**
• **Neyens, Guido, Jacobus**
**3680 Opoeteren (BE)**
• **Beyens, Dries**
**3640 Kinrooi (BE)**

(74) Vertreter: **Heraeus IP**
**Heraeus Holding GmbH**
**Intellectual Property**
**Heraeusstraße 12-14**
**63450 Hanau (DE)**

(56) Entgegenhaltungen:
EP-A1- 1 596 197      WO-A1-93/04804
DE-C1- 4 303 687      US-A- 3 315 529
US-A- 6 113 669

## Beschreibung

Die Erfindung betrifft einen Probennehmer für die Probennahme aus Schmelzen mit einem Schmelzpunkt größer 600 °Celsius, insbesondere für Metall- oder Kryolithschmelzen, insbesondere Eisen- oder Stahlschmelzen, mit einem ein Eintauchende aufweisenden Trägerrohr und einer am Eintauchende des Trägerrohres angeordneten, eine Einlauföffnung und einen Probenraum für die Schmelze aufweisenden Probenkammeranordnung, die mindestens teilweise im Inneren des Trägerrohres angeordnet ist. Des Weiteren betrifft die Erfindung eine Probenkammeranordnung für einen solchen Probennehmer sowie ein Verfahren zur Probennahme mit einem derartigen Probennehmer.

Probennehmer für Schmelzen, insbesondere für Metall- oder Kryolithschmelzen sind zahlreich bekannt. Beispielsweise in DE 32 33 677 C1 ist ein Probennehmer für Metall und Schlacke beschrieben. Dabei ist am Eintauchende eines Trägerrohres eine Einlauföffnung für einen Probenraum vorgesehen, die mit einer Schutzkappe abgedeckt ist. Ähnliche Probennehmer sind aus EP 1 544 591 A2 oder aus DE 25 04 583 A1 bekannt.

Aus DE 10 2005 060 492 B3 ist eine Kombination aus einem Probennehmer und Sensoren für die Messung in Metall- oder Schlackeschmelzen bekannt. Dabei ist ein Messkopf zur Aufnahme der Sensoren offenbart, der an seinem Eintauchende zusätzlich eine Einlauföffnung für einen Probenraum aufweist. Auch die DE 43 03 687 C1 beschreibt eine Kombination aus einem Probennehmer und Sensoren, wobei eine Einlauföffnung offenbart ist, die an der Seitenwand eines Trägerrohres angeordnet ist.

Diesen Probennehmern ist gemeinsam, dass der Probenraum am Eintauchende eines zum Eintauchen in die Schmelze bestimmten Trägerrohres angeordnet sind, wobei nach der Probennahme und dem Herausziehen des Trägerrohres aus der Schmelze das Eintauchende mit dem Probenraum vom Trägerrohr abgetrennt und danach die Probe aus dem Probenraum entnommen und einer Analyseeinrichtung zugeführt wird. Weitere ähnliche Anordnungen sind zum Trägerrohre sind unter anderem in EP 143 498 A2 und US 4,893,516 beschrieben. Hier ist auch offenbart, dass das Trägerrohr zur Messung beziehungsweise Probennahme auf eine Lanze aufgesteckt wird. Die Lanze dient zur automatisierten, maschinellen oder zur manuellen Handhabung des Trägerrohres mit dem Probenraum beziehungsweise den Sensoren. Mit Hilfe der Lanze wird das Trägerrohr in die Metallschmelze eingetaucht und aus ihr wieder herausgezogen. Das Trägerrohr mit dem Probennehmer beziehungsweise der Sensorik ist ein Einwegartikel, der nach einmaliger Benutzung entsorgt wird, während die Lanze (auch Trägerlanze) mehrfach verwendet wird.

Die Lanze dient nicht nur zum Eintauchen des Trägerrohres sondern auch zur Weiterleitung von Signalen über durch die Lanze geführte Signalkabel. Die Lanze wird mittels eines sogenannten Kupplungsstückes mit dem Trägerrohr verbunden, wobei in das Kupplungsstück auch Signalkabel oder andere funktionelle Einheiten integriert sein können. Entsprechende Kontakte mit deren Hilfe eine Weiterleitung von Signalen durch eine Trägerlanze erfolgt, sind unter anderem in DE 10 2005 060 492 B3 dargestellt.

Bei den bekannten Probennehmern erfolgt die Entnahme der Probe aus dem Probenraum unmittelbar nach dem Herausziehen des Trägerrohres aus der Metallschmelze, wobei das Eintauchende des Trägerrohres und der Probenraum selbst unter Freigabe der im Probenraum befindlichen Probe zerstört werden. Dazu kann das Trägerrohr einfach auf den Hallenboden neben dem Schmelzenbehälter fallengelassen werden, so dass das beim Eintauchvorgang ohnehin beschädigte Trägerrohr, welches in der Regel aus Pappe gebildet ist, auseinander fällt und die Probe freigibt. Probenräume für heiße Schmelzen, wie Metallschmelzen, sind häufig aus zweiteiligen Probenkammeranordnungen gebildet, so dass die beiden Teile auseinander fallen und die Probe freigeben. Insbesondere bei Hochtemperaturschmelzen, wie Stahlschmelzen, ist die Probe selbst noch sehr heiß, wenn sie durch Öffnen des Probenraumes der Umgebungsluft ausgesetzt wird. Dabei kann die Oberfläche der Probe oxidieren, so dass eine Nachbearbeitung der Probe vor der Analyse erforderlich wird. Aus DE 25 04 583 A1 ist es auch bekannt, die Probenkammeranordnung durch das Schutzgehäuse herauszuziehen.

Aufgabe der vorliegenden Erfindung ist es, die bekannten Probennehmer, insbesondere für Metall- oder Kryolithschmelzen, insbesondere Eisen- oder Stahlschmelzen, zu verbessern und eine schnellere Analyse der Proben zu ermöglichen.

Die Aufgabe wird erfindungsgemäß durch die Merkmale der unabhängigen Ansprüche gelöst. Vorteilhafte Ausgestaltungen der Erfindung ergeben sich aus den abhängigen Ansprüchen.

Der Probennehmer ist insbesondere dadurch gekennzeichnet, dass die Probenkammeranordnung an einem Teil ihrer äußeren Oberfläche eine im Inneren des Trägerrohres angeordnete Kupplungsvorrichtung zur Ankopplung einer Trägerlanze aufweist und dass die Probenkammeranordnung eine innere Wand für den Probenraum (also einen den Probenraum, in dem die Probe gewonnen werden soll, unmittelbar umgebenden Teil) und eine äußere Wand aufweist, wobei die äußere Wand die innere Wand mindestens teilweise beabstandet derart umgibt, dass zwischen äußerer Wand und innerer Wand ein Hohlraum angeordnet ist. Der Hohlraum kann gasgefüllt, insbesondere inertgasgefüllt sein oder ein Vakuum enthalten. Dadurch entsteht in der Probenkammeranordnung eine Art Doppelkammer, bei der die Innere Kammer den Probenraum darstellt und die äußere, die innere Kammer umgebende Kammer einen Gasraum (oder Vakuumraum) bildet.

Dabei ist es möglich, die Probenkammeranordnung insgesamt oder einen Teil davon mit der Trägerlanze aus dem dem Eintauchende abgewandten Ende des Trägerrohres herauszuziehen, so dass eine Oberfläche der Probe frei und der Analyse zugänglich wird. Da diese Freigabe der Oberfläche entweder in dem Trägerrohr oder bei Entnahme der gesamten Probenkammeranordnung durch das Trägerrohr hindurch nach außen erfolgen kann, ist die Probe unter kontrollierten Bedingungen einer Analyse zuführbar. Dabei erfolgt die Entnahme der Probe oder Freigabe einer Probenoberfläche derart, dass sie der Analyse unmittelbar und ohne vorherige Präparation zugeführt werden kann. Unter dem Begriff Probenanalyse ist in diesem Zusammenhang die Aufnahme der für die Auswertung notwendigen optischen Signale durch ein Spektrometer (und deren Auswertung) zu verstehen; von dort erfolgt eine Weiterleitung der Signale an Auswerteeinrichtungen, Computer oder Ähnliches.

Insbesondere ist es vorteilhaft, dass die Probenkammeranordnung als innere Wand mehrere, den Probenraum unmittelbar umgebende und von einander lösbare Teile aufweist, wobei mindestens eines der Teile im Inneren des Trägerrohres angeordnet ist. Dadurch kann zumindest dieses Teil mittels der Trägerlanze durch das Trägerrohr hindurch von der Probe entfernt werden. Zweckmäßig ist es weiterhin, dass die Probenkammeranordnung oder der die Kupplungsvorrichtung aufweisende Teil der Probenkammeranordnung am Eintauchende des Trägerrohres derart angeordnet ist, dass die Probenkammeranordnung oder der die Kupplungsvorrichtung aufweisende Teil durch das Innere des Trägerrohres hindurch zu dem dem Eintauchende abgewandten Ende des Trägerrohres und dort aus dem Trägerrohr heraus bewegbar ist.

Bevorzugt weist die Probenkammeranordnung oder der die Kupplungsvorrichtung aufweisende Teil der Probenkammeranordnung einen Querschnitt senkrecht zur Längsachse des Trägerrohres auf, der höchstens so groß ist wie der Querschnitt des Inneren des Trägerrohres senkrecht zu seiner Längsachse. Der Querschnitt der Probenkammeranordnung beziehungsweise des die Kupplungsvorrichtung aufweisenden Teils der Probenkammeranordnung ist dabei durch seine Silhouette (Umrisskante) charakterisiert, die an keiner Stelle über den Querschnitt des Inneren des Trägerrohres hinausragen darf, da sonst eine Entnahme der Probenkammeranordnung beziehungsweise ihres Teils durch das Trägerrohr nicht oder nur nach einem Kippen gegen die Längsachse möglich ist, was den Erfolg entweder verhindert oder nur bei erhöhten Aufwand ermöglicht.

Zweckmäßig ist es, dass die Probenkammeranordnung ein erstes Teil und ein zweites Teil aufweist, die gemeinsam den Probenraum umgeben, dass das Trägerrohr ein die Kupplungsvorrichtung enthaltendes Hauptteil und ein am Eintauchende des Trägerrohres vom Hauptteil lösbar angeordnetes Endteil aufweist, wobei das erste Teil der Probenkammeranordnung an dem Hauptteil und das zweite Teil der Probenkammeranordnung an dem Endteil des Trägerrohres fixiert ist. Dadurch ist es möglich, das Trägerrohr nach der Probennahme auf einfache und schnelle Weise auseinanderzunehmen, wobei gleichzeitig die Teile der Probenkammeranordnung voneinander gelöst werden, so dass ein Teil der Probenoberfläche für eine Analyse frei zugänglich ist. Das Trägerrohr muss nicht mit großem Aufwand auseinandergebrochen oder geschnitten werden, da es eine Sollbruchstelle an der Verbindungsstelle von Hauptteil und Endteil aufweist.

Das Endteil kann mittels einer Steck- oder Schraubverbindung mit dem Hauptteil verbunden sein. Vorzugsweise kann das Endteil mittels Klammern oder Krampen mit dem Hauptteil verbunden sein. Dadurch ist einerseits eine sichere Verbindung für den Transport und den Gebrauch des Probennehmers gewährleistet bei andererseits leichter und schneller Trennungsmöglichkeit durch einfache Handgriffe oder Werkzeuge. Wenn der Probennehmer an seinem Eintauchende zusätzlich Sensoren, wie beispielsweise ein Thermoelement oder einen elektrochemischen Sensor aufweist, sind Anschlußdrähte dieser Sensoren durch das Innere des Trägerrohres verlegt. Diese Anschlußdrähte werden beim Trennen des Endteils vom Hauptteil des Trägerrohres ebenfalls getrennt. Dabei können Sollbruchstellen oder übliche Trennmechanismen vorgesehen sein, die die Anschlußdrähte unterhalb der freizusetzenden Probenoberfläche, also zwischen Probenoberfläche und Eintauchende des Probennehmers, trennen. Dadurch wird vermieden, dass die Anschlußdrähte eventuell auf oder über die zu analysierende Probenoberfläche gelangen und die Analyse stöhren.

Weiterhin ist es vorteilhaft, dass die Kupplungsvorrichtung als Rastkupplung oder als Bajonettkupplung oder als Schraubkupplung ausgebildet ist, um ein einfaches Ankoppeln an übliche Trägerlanzen zu ermöglichen. Zweckmäßig ist es weiterhin, dass das Verhältnis der Masse der in dem Probenraum aufgenommen Schmelze zur Masse der Probenkammeranordnung, insbesondere der inneren Wand der Probenkammeranordnung, ohne Schmelze kleiner als 0,8, vorzugsweise höchstens 0,1 ist. Dadurch wird ein schnelles Abkühlen der in den Probenraum eingelaufenen Schmelze erreicht, so dass diese bei Freigabe einer Probenoberfläche bereits soweit abgekühlt ist, dass eine Oxidation weitestgehend verhindert und damit eine Nachbearbeitung der Probenoberfläche unnötig wird.

Die Probenkammeranordnung weist eine innere Wand für den Probenraum und eine äußere Wand auf, wobei die äußere Wand die innere Wand mindestens teilweise beabstandet unter Bildung eines Hohlraumes umgibt. Das Masseverhältnis kann mit Hilfe des Materials der inneren Wand für den Probenraum gebildet werden. Die innere Wand der Probenkammeranordnung kann ganz oder teilweise aus Kupfer gebildet sein, um den Kühleffekt zu erhöhen. Durch die teilweise Beabstandung der äußeren von der inneren Wand kann in den entstehenden Hohlraum ein Inertgas eingeführt werden, um eine nicht oxidierende Atmosphäre in unmittelbare Probenumgebung zu schaffen. Das Inertgas kann vorzugsweise dadurch zugeführt werden, dass die Kupplungsvorrichtung mindestens einen Gasdurchlaufkanal aufweist, der durch die äußere Wand der Probenkammeranordnung hindurch oder an diese heranführt.

Eine erfindungsgemäße Probenkammeranordnung für einen oben beschriebenen Probennehmer, mit einem Probenraum, der von einer aus mehreren Teilen gebildeten inneren Wand unmittelbar umgeben ist und einem mit dem Probenraum verbundenen Einlaufrohr zur Aufnahme einer Probe aus einer Metall- oder Kryolithschmelze, insbesondere einer Stahlschmelze in dem Probenraum, wobei das Einlaufrohr mit einer Einauföffnung in den Probenraum mündet, ist dadurch gekennzeichnet, dass das Verhältnis V zwischen Masse M (g) der Probe und dem Material der inneren Wand durch folgendes Verhältnis charakterisiert ist:

$$V = \frac{M \times 24000}{m \times c \times \lambda} < 0{,}15 \ ,$$

wobei m die Masse (g) der inneren Wand, c die spezifische Wärmekapazität (J/kg.K) und $\lambda$ die thermische Leitfähigkeit (W/m.K) des Materials der inneren Wand sind. Vorzugsweise ist das Verhältnis V < 0,05. Dadurch wird eine schnelle Abkühlung der einlaufenden Schmelze von beispielsweise mehr als 1600 °C (bei Stahlschmelze) auf 200 bis 300 °C bewirkt, so dass die Oberfläche der Analyse zugeführt werden kann, ohne zu oxidieren. Die Probe kann insbesondere eine Stahl- oder Eisenprobe sein, als Material der inneren Wand ist unter anderem Kupfer oder Aluminium geeignet. Auch mit einem Verhältnis V < 0,3 können ggf. noch gute Proben erzielt werden.

Die Probenkammeranordnung weist durch ihre beiden Teile der inneren Wand eine flache Form auf (zum Beispiel als kreisförmige Scheibe) mit einer nach Entfernen des einen Teils der inneren Wand freiliegenden glatten, ebenen Probenoberfläche. Der zweite Teil der inneren Wand, in dem die Probe zunächst verbleibt, kann Öffnungen aufweisen, durch die bei Füllen des Probenraums Gas entweichen oder vorher Gas abgesaugt und ein Vakuum gebildet werden und ggf. vorhandener Überschuß an einlaufender Schmelze austreten kann. Die durch die Öffnungen ausgetretene Schmelze erstarrt wie die Probe selbst und fixiert die Probe an diesem zweiten Teil der inneren Wand, so dass sie leichter der Analyse zugeführt werden kann. Auch der erste Teil der inneren Wand kann Öffnungen für die Entlüftung des Probenraumes aufweisen.

Das Verhältnis von Probenvolumen, also Volumen (in mm³) des Probenraumes zu dem Gesamtquerschnitt (in mm²) der der Entlüftung dienenden, aus dem Probenraum herausführenden Öffnungen ist kleiner als 500 mm, vorzugsweise kleiner als 100 mm, um eine effektive Entlüftung zu ermöglichen.

Die Teile der Wand der Probenkammeranordnung können mit Federn, insbesondere Spiralfedern gegeneinandergepresst und so zusammengehalten werden.

Die Probenkammeranordnung weist vorteilhaft mindestens zwei den Probenraum unmittelbar umgebende und voneinander lösbare Teile auf.

Vorzugsweise weist das Einlaufrohr an der Einlauföffnung einen verringerten Querschnitt auf. Es ist also als Rohr mit einen etwa gleichen Durchmesser und Querschnitt über seine Länge ausgebildet, wobei sich der Querschnitt an der Einlauföffnung verringert. Die entgegengesetzte Öffnung zum Einlaufen der Schmelze in das Einlaufrohr kann ebenfalls einen verkleinerten Querschnitt aufweisen, der bevorzugt kleiner ist als der Querschnitt der Einlauföffnung in den Probenraum. Dabei ist bevorzugt der Querschnitt des Einlaufrohres kreisförmig und die entgegengesetzte Öffnung kann einen Durchmesser von ca. 3 mm aufweisen, während das Einlaufrohr einen Durchmesser von ca. 8 mm über seine Länge aufweisen kann und die Einlauföffnung in den Probenraum einen Durchmesser von ca. 6 mm.

Das erfindungsgemäße Verfahren zur Probennahme aus Schmelzen mit einer Schmelztemperatur von größer 600 °Celsius, insbesondere für Metall- oder Kryolithschmelzen, insbesondere für Eisen- oder Stahlschmelzen, mit einem erfindungsgemäßen Probennehmer, wobei durch das einem Eintauchende des Trägerrohres entgegengesetzten Ende hindurch eine Trägerlanze in das Trägerrohr eingeschoben wird, ist dadurch gekennzeichnet, dass die Trägerlanze an die Kupplungsvorrichtung der Probenkammeranordnung angekoppelt wird, dass danach das Eintauchende des Trägerrohres in die Schmelze eingetaucht und der Probenraum der Probenkammeranordnung mit Schmelze gefüllt wird, dass anschließend die Probenkammeranordnung oder der die Kupplungsvorrichtung aufweisende Teil der Probenkammeranordnung mit der Trägerlanze durch das Trägerrohr hindurch aus dem dem Eintauchende entgegengesetzten Ende des Trägerrohres herausgezogen wird und dass nach dem Herausziehen des die Kupplungsvorrichtung aufweisenden Teils der Probenkammeranordnung aus dem Trägerrohr, wobei ein Teil der Oberfläche der in der Probenkammeranordnung befindlichen Probe in unmittelbaren Kontakt mit der Umgebung der Probenkammeranordnung (oder mit dem zwischen der inneren Wand und der äußeren Wand der Probenkammeranordnung angeordneten Hohlraum) tritt, eine ein Spektrometer aufweisende Lanze in das Trägerrohr eingeschoben und die Oberfläche der Probe mit Hilfe des Spektrometers analysiert wird.

Eine alternative Ausführungsform des erfindungsgemäße Verfahrens zur Probennahme aus Schmelzen mit einer Schmelztemperatur von größer 600 °Celsius, insbesondere für Metall- oder Kryolithschmelzen, insbesondere für Eisen- oder Stahlschmelzen, mit einem erfindungsgemäßen Probennehmer, wobei durch das einem Eintauchende des Trägerrohres entgegengesetzten Ende hindurch eine Trägerlanze in das Trägerrohr eingeschoben wird, ist dadurch gekennzeichnet, dass die Trägerlanze an die Kupplungsvorrichtung der Probenkammeranordnung angekoppelt wird, dass danach das Eintauchende des Trägerrohres in die Schmelze eingetaucht und der Probenraum der Probenkammeranordnung mit Schmelze gefüllt wird, dass anschließend die Probenkammeranordnung oder der die Kupplungsvorrichtung aufweisende Teil der Probenkammeranordnung mit der Trägerlanze durch das Trägerrohr hindurch aus dem dem Eintauchende entgegengesetzten Ende des Trägerrohres herausgezogen wird und dass nach dem Herausziehen des die Kupplungsvorrichtung aufweisenden Teils der Probenkammeranordnung aus dem Trägerrohr, wobei ein Teil der Oberfläche der in der Probenkammeranordnung befindlichen Probe in unmittelbaren Kontakt mit der Umgebung der Probenkammeranordnung (oder mit dem zwischen der inneren Wand und der äußeren Wand der Probenkammeranordnung angeordneten Hohlraum) tritt, eine einen Greifer oder Manipulator aufweisende Lanze in das Trägerrohr eingeschoben wird, der Greifer oder Manipulator die Probe ergreift, diese aus der Probenkammeranordnung entnimmt und durch das Trägerrohr hindurch aus dem dem Eintauchende entgegengesetzten Ende des Trägerrohres herauszieht.

Dadurch verbleibt die Probe relativ lange in einer vergleichsweise gut geschützten Umgebung und kann dort abkühlen bis auf eine Temperatur, bei der die Probenoberfläche nicht sofort oxidiert, so dass sie ohne weitere Bearbeitung analysiert werden kann. Gegenüber Schmelzentemperaturen von Eisen- oder Stahlschmelzen von mehr als 1450 °Celsius sind dies nur noch wenige hundert °Celsius, beispielsweise 200 - 300 °Celsius. Dabei erfolgt eine Analyse in einer relativ geschützten Umgebung, ohne dass die Probe selbst aus dem Trägerrohr entfernt werden muss. Dies kann insbesondere dann vorteilhaft sein, wenn der nicht mit der Trägerlanze entfernte Teil der Probenkammeranordnung aus unterschiedlichen Gründen an dem Eintauchende des Trägerrohres derart befestigt ist, dass er nicht ohne Zerstörung des Trägerrohres von diesem entfernt werden kann.

Vorzugsweise wird die Probenkammer nach dem Herausziehen aus dem Trägerrohr einer Analyseneinrichtung zugeführt.

Daneben kann es vorteilhaft sein, dass nach dem Herausziehen des die Kupplungsvorrichtung aufweisenden Teils der Probenkammeranordnung aus dem Trägerrohr, wobei ein Teil der Oberfläche der in der Probenkammeranordnung befindlichen Probe in unmittelbaren Kontakt mit der Umgebung der Probenkammeranordnung (oder mit dem zwischen der inneren Wand und der äußeren Wand der Probenkammeranordnung angeordneten Hohlraum) tritt, die Probe aus dem Eintauchende des Trägerrohres entfernt wird, wobei sie entweder durch das Eintauchende hindurch herausgezogen oder das Eintauchende oder ein Teil davon mit der Probe aus dem Trägerrohr entfernt wird. Dies kann sinnvoll sein, wenn aufgrund baulicher oder betrieblicher Eigenarten eine Analyse innerhalb des Trägerrohres oder einer Entnahme der Probe durch das Trägerrohr hindurch nicht möglich oder sinnvoll ist. Es ist vorteilhaft, dass die Probe außerhalb des Trägerrohres einer Analyseneinrichtung zugeführt wird.

Vorteilhaft ist es weiterhin, dass das Trägerrohr vor dem Herausziehen der Probenkammeranordnung oder des die Kupplungsvorrichtung aufweisenden Teils der Probenkammeranordnung aus dem Trägerrohr aus der Schmelze gezogen wird. Des Weiteren kann es sinnvoll sein, dass während und/oder nach der Probennahme an die Probenkammeranordnung oder in einen Hohlraum der Probenkammeranordnung ein Inertgas geleitet wird. Dadurch wird Sauerstoffzutritt zur Probe verhindert, so dass diese auch bei hohen Temperaturen nicht oxidieren kann und eine Analyse ohne weitere Nachbearbeitung der Probe möglich ist.

Nachfolgend wird die Erfindung beispielhaft anhand von Zeichnungen erläutert. In den Zeichnungen zeigt

| | |
|---|---|
| Figur 1 | eine schematische Darstellung eines erfindungsgemäßen Probennehmers |
| Figur 2 | Teile des Probennehmers nach der Probenentnahme |
| Figur 3 | Teile des Probennehmers vor einer Analyse |
| Figur 4 | Teile der Probenkammer |
| Figur 5 | eine Analyse innerhalb des Trägerrohres |
| Figur 6 | eine Entnahme der Probe innerhalb des Trägerrohres |
| Figur 7 | die Vorbereitung einer Analyse durch Abtrennung des Eintauchendes des Probennehmers |
| Figur 8 | einen weiteren erfindungsgemäßen Probennehmer |
| Figur 9 | Teile des weiteren Probennehmers bei der Entformung der Probe |
| Figur 10 | das Oberteil der inneren Wand der Probenkammeranordnung |
| Figur 11 | das Unterteil der inneren Wand der Probenkammeranordnung |
| Figur 12 | die Probenkammeranordnung mit innerer und äußerer Wand |
| Figur 13 | die entformte Probenkammeranordnung mit innerer und äußerer Wand. |

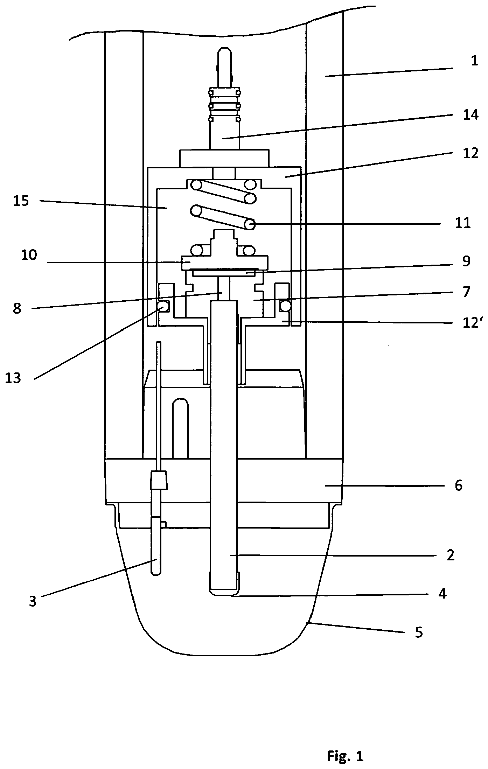

In Figur 1 ist eine Schnittdarstellung des Eintauchendes des Probennehmers gezeigt. Am Eintauchende des Trägerrohres 1 mündet das Einlaufrohr 2 für die Metallschmelzenprobe sowie ein Thermoelement 3. Das Einlaufrohr 2 ist mit einer Metallkappe 4 geschützt. Diese sowie die äußere Schutzkappe 5 aus Metall verhindern Transportschäden und Schäden beim Durchdringen des Probennehmers durch eine Schlackenschicht, bevor die Öffnung des Einlaufrohres 2 nach Eintauchen in eine Metallschmelze, beispielsweise eine Stahlschmelze, freigegeben wird. Thermoelement 3 und Einlaufrohr 2 sind in einem feuerfesten Körper 6 fixiert.

Das Einlaufrohr 2 mündet in einem Kühlkörper 7, durch dessen Durchlass 8 die Metallschmelze in den Probenraum 9 dringt. Dieser wird an seiner (in Eintauchrichtung gesehen) oberen Seite von einem oberen Kühlkörper 10 geschlossen. Die Kühlkörper 7; 10 können aus Kupfer gebildet sein, sodass ein schneller Wärmeabfluss aus der eingelaufenen Probe erfolgt und diese schnell abgekühlt wird. Der untere Kühlkörper 7 und der obere Kühlkörper 10 bilden zusammen die innere Wand der Probenkammeranordnung. Der Probenraum 9 selbst weist eine Dicke von etwa 2 mm und einen Durchmesser von etwa 28 mm auf.

Vorzugsweise ist das Verhältnis der Masse der in den Probenraum 9 eingelaufenen Stahlschmelze zu der Masse der Kühlkörper 7; 10 kleiner als 0,1, sodass die Metallschmelze sehr schnell erstarrt und auf eine Temperatur von etwa 200 °Celsius abkühlt. Die Kühlkörper 7; 10 sind aus Kupfer gebildet. Dabei ergibt sich bei der in den Figuren dargestellten verhältnismäßigen Größe ein Verhältnis V von etwa 0,0167. Der obere Kühlkörper 10 wird mittels einer Spiralfeder 11 gegen den unteren Kühlkörper 7 gedrückt, sodass der Probenraum 9 abgedichtet ist. Das Gegenlager der Spiralfeder 11 wird durch die äußere Wand 12; 12' der Probenkammeranordnung gebildet. Der untere Teil 12' der äußeren Wand weist einen Durchlass für das Einlaufrohr 2 auf und bildet das untere Gegenlager für die Spiralfeder 11 sowie die Kühlkörper 7; 10. Die Kühlkörper 7; 10 bilden die innere Wand der Probenkammeranordnung. Die beiden Teile der äußeren Wand 12; 12' sind miteinander fixiert und mittels einer Dichtung 13 abgedichtet.

An der Oberseite des oberen Teils 12 der äußeren Wand ist eine Kupplungsvorrichtung 14 für eine in der Zeichnung nicht dargestellte Trägerlanze angeordnet. Die Kupplungsvorrichtung 14 ist als Rastkupplung ausgebildet, sodass die Trägerlanze an ihr fixiert und nach der Probennahme der obere Teil 12 der äußeren Wand mit dem oberen Kühlkörper 10 oder auch die gesamte Probenkammeranordnung einschließlich des unteren Teils 12' der äußeren Wand und des unteren Kühlkörpers 7 durch das Trägerrohr 1 hindurch nach oben herausgezogen werden kann. Beim Herausziehen der kompletten Probenkammeranordnung kann das Einlaufrohr 2, welches im unteren Teil der Probenkammeranordnung fixiert ist, ebenfalls herausgezogen werden.

Die Kupplungsvorrichtung 14 weist einen im Wesentlichen in ihrer Längsachse verlaufenden Gasdurchlaufkanal auf, der über die Trägerlanze mit einer Inertgasquelle verbunden werden kann und durch den Inertgas in den Hohlraum 15 der Probenkammeranordnung eingeleitet werden kann, sodass nach Entfernen des oberen Kühlkörpers 7 von dem Probenraum 9 die Probe von Inertgas umgeben ist und nicht oxidieren kann. Das Thermoelement 3 weist an seinem dem Eintauchende abgewandten und nur schematisch dargestellten Ende in bekannter Weise einen elektrischen Verbinder auf, der in ebenfalls bekannter Weise mit einer Trägerlanze verbunden werden kann, sodass die elektrischen Signale des Thermoelementes nach außen zu einer Auswerteeinrichtung geleitet werden können.

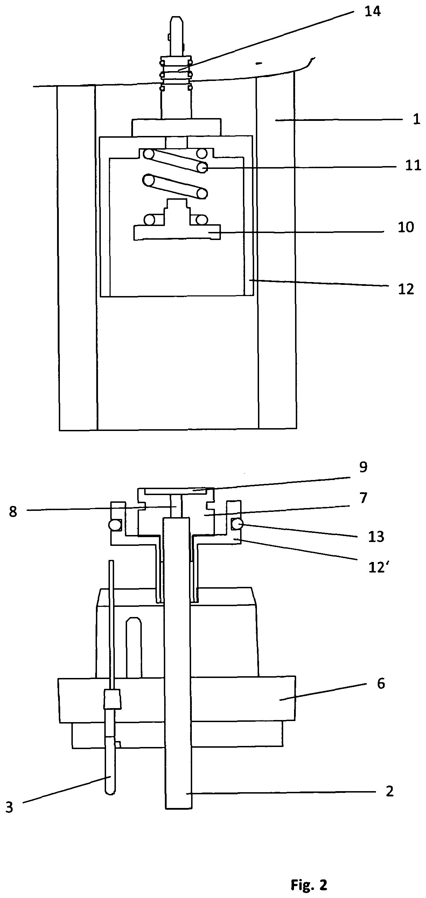

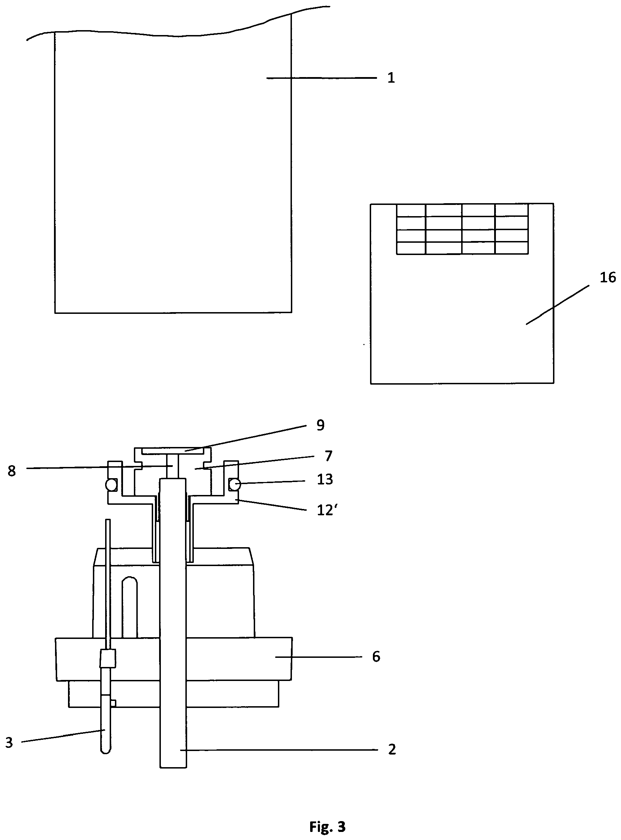

In Figur 2 ist das Entfernen des oberen Teils der Probenkammeranordnung mit dem oberen Teil 12 der äußeren Wand, dem oberen Kühlkörper 10 und der Spiralfeder 11 von dem unteren Teil der Probenkammeranordnung mit dem mit Metallschmelze gefüllten Probenraum 9, dem unteren Kühlkörper 7 und dem unteren Teil 12' der äußeren Wand dargestellt. Nach dem Trennen dieses unteren Teils vom oberen Teil der Probenkammeranordnung wird der untere Teil aus dem Eintauchende des Trägerrohres 1 herausgezogen, sodass die im Probenraum 9 angeordnete und inzwischen erkaltete Probe einem Spektrometer 16 (siehe Figur 3) zur Analyse zugeführt werden kann.

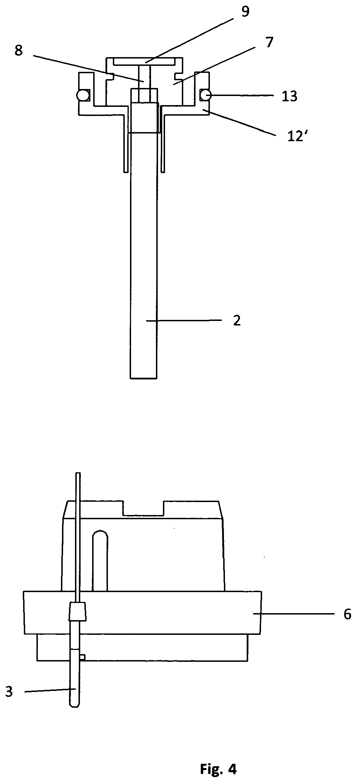

In Figur 4 ist die Entnahme des unteren Teils der Probenkammeranordnung einschließlich des Einlaufrohres 2 aus dem feuerfesten Körper 6 dargestellt.

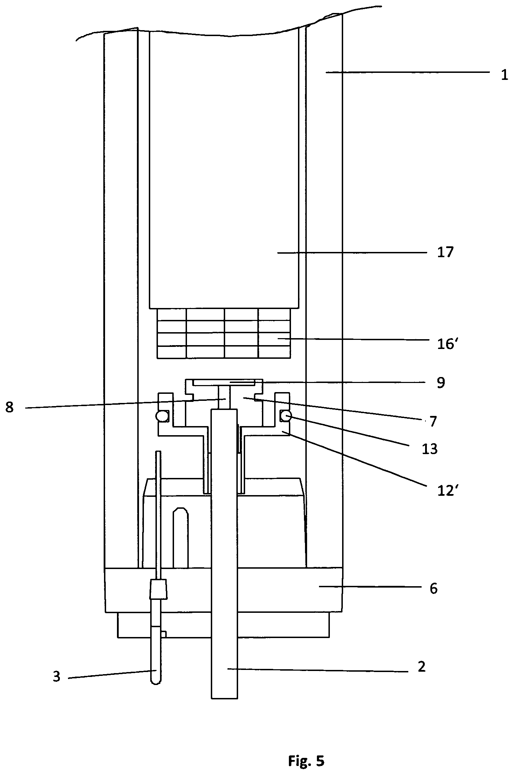

Die Entfernung des oberen Teils der Probenkammeranordnung durch die Trägerlanze 1 hindurch ist in Figur 5 dargestellt. Hier ist gezeigt, dass nach der Entfernung des oberen Teils der Probenkammeranordnung und der Freigabe der Oberfläche der Probe in dem Probenraum 9 ein Spektrometer 16' mit Hilfe einer Spektrometerlanze 17 durch das Trägerrohr 1 hindurch an den Probenraum 9 herangeführt ist, sodass die Probenanalyse innerhalb des Trägerrohres 1 erfolgen kann. Unter dem Begriff Probenanalyse ist in diesem Zusammenhang die Aufnahme der für die Auswertung notwendigen optischen Signale durch das Spektrometer 16; 16' zu verstehen, von dort erfolgt eine Weiterleitung der Signale an Auswerteeinrichtungen, Computer oder Ähnliches.

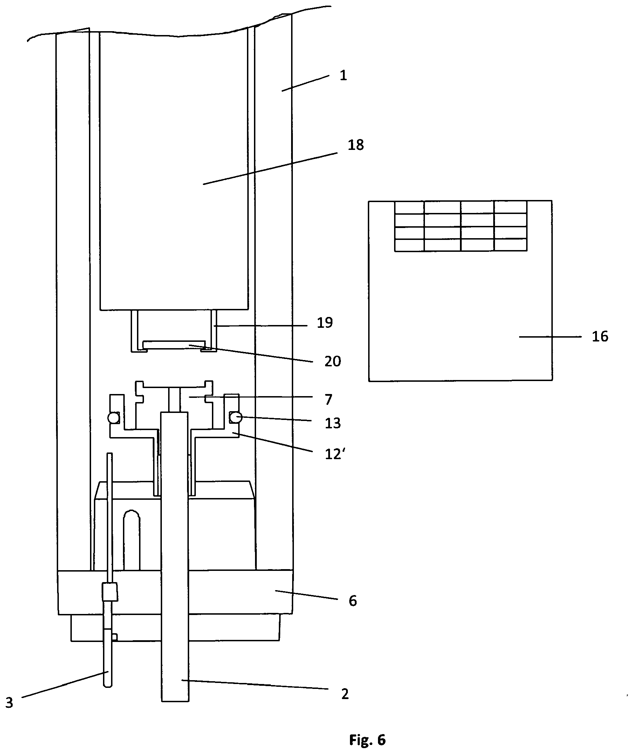

Figur 6 zeigt eine weitere Möglichkeit der Probenanalyse. Hier wird im Unterschied zu der in Figur 5 dargestellten Variante nach der Entfernung des oberen Teils der Probenkammeranordnung eine Greiferlanze 18 in das Trägerrohr eingeführt, mit deren Hilfe ein Greifer 19 die Probe 20 erfassen und durch das Trägerrohr 1 hindurch herausziehen kann, sodass die Probe 20 mit Hilfe der Greiferlanze 18 dem Spektrometer 16 zugeführt werden kann.

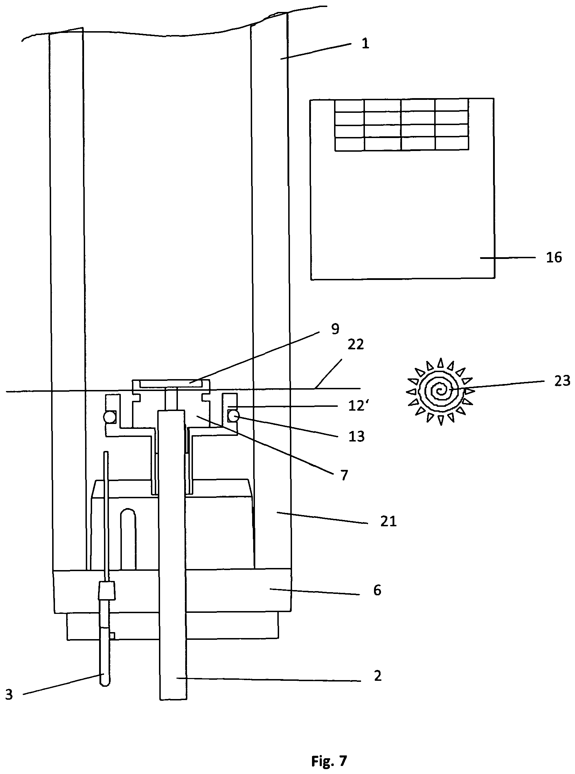

Eine Alternative zu der in Figur 2 dargestellten Ausführungsform ist in Figur 7 angegeben. Statt den unteren Teil der Probenkammeranordnung mit dem feuerfesten Körper 6 aus dem Eintauchende des Trägerrohres nach unten herauszuziehen, kann nach Entfernen des oberen Teils der Probenkammeranordnung mittels der Trägerlanze durch das Trägerrohr 1 hindurch das Eintauchende 21 des Trägerrohres 1 etwa in Höhe des Probenraumes 9 längs der Markierung 22 von dem übrigen Teil des Trägerrohres 1 abgetrennt werden. Dazu kann ein übliches Schneidwerkzeug 23, das in Figur 7 lediglich schematisch angedeutet ist, verwendet werden. Auch in diesem Fall kann die Probe anschließend einem Spektrometer 16 zur Analyse zugeführt werden.

Statt das Eintauchende abzuschneiden kann dieses auch auf andere herkömmliche Weise derart zerstört werden, dass die Probe entnommen und der Analyse zugeführt werden kann. Auch in diesem Fall hat die Probe vor der Entnahme aus dem Trägerrohr 1 durch die Kühlkörper 7; 10 eine ausreichende Kühlung erfahren. Sie ist nach Abtrennen des Eintauchendes 21 und Entnahme des Unterteils der Probenkammer ohne Weiteres der Analyse zugänglich, wobei die Freisetzung der zu analysierenden Probenoberfläche in einer "sauberen" und gegebenenfalls inerten Atmosphäre erfolgt ist.

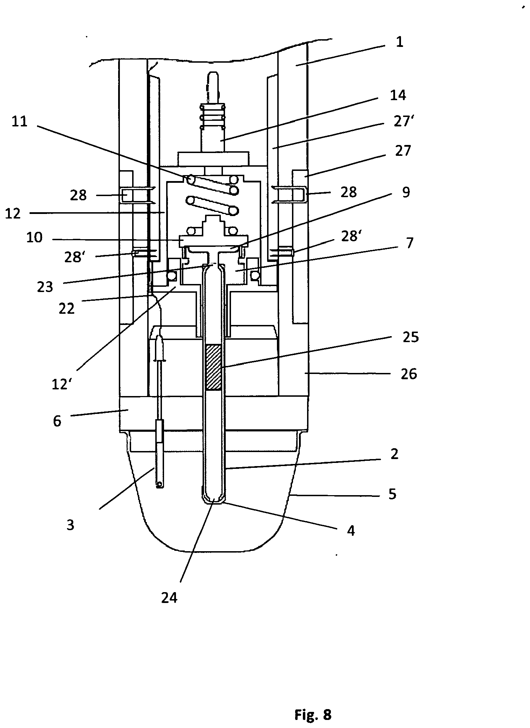

Figur 8 stellt eine zu Figur 1 alternative oder bevorzugte Ausführung des Probennehmers dar. Die Probenkammeranordnung im Inneren des Probennehmers ist analog zu der in Figur 1 gezeigten Ausführungsform aufgebaut. Die Kühlkörper 7; 10 sind aus Kupfer gebildet. Dabei ergibt sich bei der in den Figuren dargestellten verhältnismäßigen Größe ein Verhältnis V von etwa 0,0167.

Das Einlaufrohr 2 weist einen Durchmesser von etwa 8 Millimeter auf, seine Einlauföffnung 23 hat einen Durchmesser von etwa 6 Millimeter und die entgegengesetzte Öffnung 24 weist einen Durchmesser von etwa 3 Millimeter auf. In dem Einlaufrohr 2 ist Aluminium als Deoxidationsmittel 25 angeordnet. Neben dem Einlaufrohr 2 ist an dem feuerfesten Körper 6 ein Thermoelement 3 fixiert, welches mittels Thermoelementdrähten 22 mit einer Messelektronik verbunden ist.

Das Trägerrohr 1 aus Pappe weist an seinem Eintauchende ein Papprohr 26 auf. Dieses ist mit dem Trägerrohr 1 durch ein die aneinander stoßenden Enden des Trägerrohres 1 und des Papprohres 26 verbindendes Verbindungsrohr 27 fixiert. Das Verbindungsrohr 27 schließt an seinem äußeren Umfang etwa bündig mit dem Trägerrohr 1 und dem Papprohr 26 ab. Im Inneren des Trägerrohres 1 ist ein inneres Verbindungsrohr 27' angeordnet, das die Verbindungsstelle zwischen dem Papprohr 26 und dem Trägerrohr 1 überbrückt und an seiner Innenseite an dem oberen Teil der äußeren Wand 12 der Probenkammer anliegt und dieses fixiert.

Trägerrohr 1, Papprohr 26 und die Verbindungsrohre 27; 27' sind mit Klammern 28; 28' miteinander verbunden. Diese Verbindungsstelle ist leicht durch einfache mechanische Einwirkung zu lösen, so dass das Trägerrohr 1 von dem Papprohr 26 entfernt werden kann, wobei das mittels des inneren Verbindungsrohres 27' mit dem Trägerrohr 1 verbundene obere Teil der äußeren Wand 12 der Probenkammeranordnung an dem Trägerrohr 1 verbleibt und das untere Teil der äußeren Wand 12' an dem Papprohr 26 fixiert ist und an diesem verbleibt, so dass beide Teile der äußeren Wand 12; 12' voneinander gelöst werden.

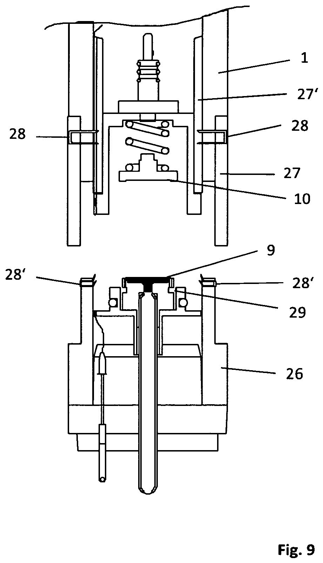

Das Verbindungsrohr 27 ist dabei mit einer größeren Klammer 28 mit dem Trägerrohr 1 und dem inneren Verbindungsrohr 27' verbunden, während das Papprohr 26 mit dem inneren Verbindungsrohr 27' mit kleineren Klammern 28' verbunden ist. Diese Verbindung mit den kleineren Klammern 28' wird beispielsweise bei einer Drehung von Trägerrohr 1 gegen Papprohr 26 gelöst, wie in Figur 9 gezeigt. Dabei werden auch die Anschlußdrahte von Sensoren, also die Thermoelementdrähte 22 durchgetrennt. Diese Trennung erfolgt, wie im Figur 9 beispielhaft dargestellt, unterhalb der bei der Trennung freigelegten Analysenoberfläche der Probe, also zwischen der Analysenoberfläche und dem Eintauchende des Probennehmers, so dass die durchtrennten Thermoelementdrähte nicht vor die Analysenoberfläche gelangen und die Analyse stören können.

Der Probenraum 9 mit der Probe ist nach der Trennung nunmehr einer Analyse zugänglich, da der Kühlkörper 10 entfernt wurde und eine ebene, glatte Probenoberfläche freigibt. Die in dem unteren Kühlkörper 7 angeordneten Öffnungen 29 verbinden den Probenraum mit dem zwischen der durch Kühlkörper 7 der inneren Wand und dem unteren Teil 12' der äußeren Wand gebildeten Hohlraum 15. Durch diese Öffnungen 29, die gleichmäßig kreisförmig angeordnet in den Umfang des Probenraumes münden, kann überschüssige Schmelze aus dem Probenraum austreten, so dass lunkerfreie Proben gewonnen werden. Die ausgetretene Schmelze erstarrt und hält so die Probe in diesem Teil der Probenkammeranordnung fest, so dass sie mit der Probenkammeranordnung beziehungsweise einem Teil der Probenkammeranordnung, nämlich dem dem Eintauchende zugewandten, unteren Teil, einer Analyse zugeführt werden kann.

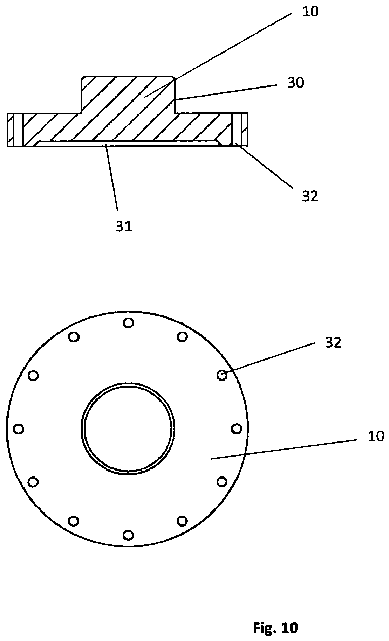

In Figur 10 ist der obere Kühlkörper 10 im Querschnitt und darunter in der Draufsicht gezeigt. An seiner oberen Seite ist eine Erhöhung mit einer Flanke 30 angeordnet. An der Flanke 30 wird die Spiralfeder 11 fixiert. Gegenüberliegend an der unteren Seite des oberen Kühlkörpers 10 ist das Oberteil 31 des Probenraumes 9 dargestellt. Seitliche, kreisförmige Ventilationsöffnungen 32 dienen der Gaszirkulation oder der gezielten Gaszu- oder abfuhr in dem Hohlraum 15.

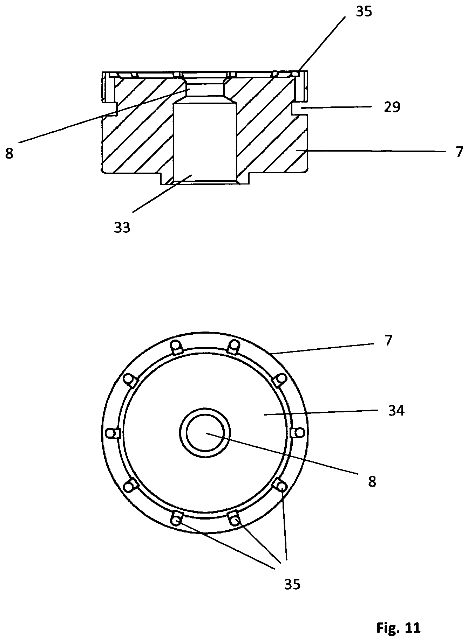

Figur 11 zeigt den unteren Kühlkörper 7 im Querschnitt und darunter in der Draufsicht. In dem unteren Kühlkörper sind der Durchlass 8 für Metallschmelzen sowie die Aufnahme 33 für das Einlaufrohr 2 angeordnet. Durch Ventilationsöffnungen 35 können Gase vor oder während der Probennahme aus dem Probenraum 9 entweichen. Die Ventilationsöffnungen 35 sind mit den Öffnungen 29 verbunden, durch die überschüssiges Metall aus dem Unterteil 34 des Probenraumes austreten kann.

Das Verhältnis von Volumen (in $mm^3$) des Probenraumes zum Gesamtquerschnitt (in $mm^2$) der der Entlüftung dienenden Öffnungen (also Ventilationsöffnungen 32;35) beträgt in den in den Figuren beispielhaft dargestellten Anordnungen etwa 72 mm, wobei das Volumen des Probenraumes 9 etwa 1230 $mm^3$ und und der Gesamtquerschnitt der Ventilationsöffnungen 32;35 etwa 17 $mm^2$ beträgt.

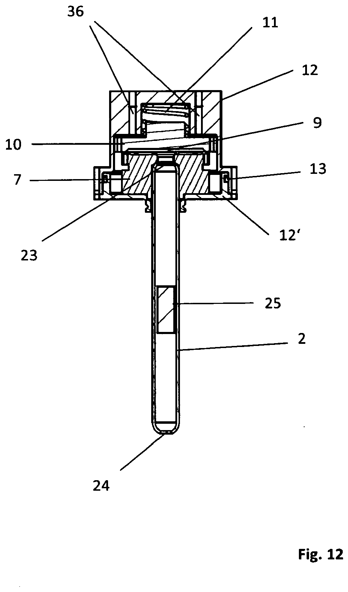

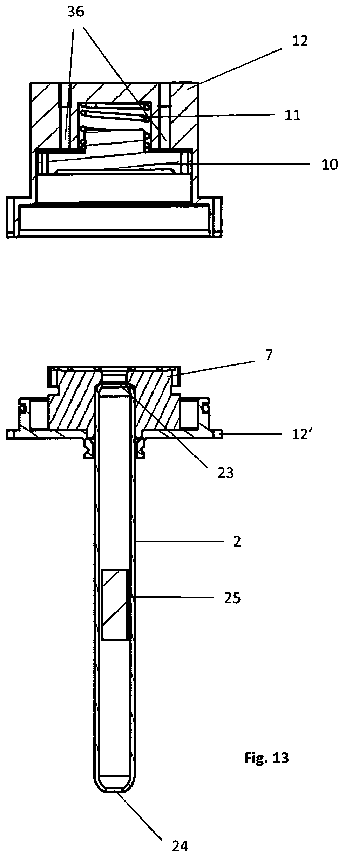

In den Figuren 12 und 13 ist die Probenkammeranordnung mit innerer und äußerer Wand dargestellt, im montierten Zustand (Figur 12) und im entformten Zustand (Figur 13). Der obere Teil 12 und der untere Teil 12' der äußeren Wand sind aus Aluminium gebildet. Sie umgreifen die Kühlkörper 7;10, die mittels einer Spiralfeder 11 zusammengedrückt werden und den Probenraum 9 umschließen. Deutlich sichtbar ist das Einlaufrohr 2 mit seinen beiden im Durchmesser verringerten Enden, der Einlauföffnung 23 und der entgegengesetzten Öffnung 24. Ebenfalls dargestellt sind die Gasduchlaufkanäle 36 desoberen Teils 12 der äußeren Wand.

Die Beispiele beschränken die Erfindung nicht, insbesondere sind funktionell nicht unmittelbar zusammenwirkende Merkmale einer Ausführungsform auch auf andere speziell oder allgemein beschriebene Ausführungsformen der Erfindung übertragbar.

**Patentansprüche**

1. Probennehmer für die Probennahme aus Schmelzen mit einem Schmelzpunkt größer 600 °C, insbesondere für Metall- oder Kryolithschmelzen, mit einem ein Eintauchende (21) aufweisenden Trägerrohr (1) und einer am Eintauchende (21) des Trägerrohres (1) angeordneten, eine Einlauföffnung und einen Probenraum (9) für die Schmelze aufweisenden Probenkammeranordnung, die mindestens teilweise im Inneren des Trägerrohres (1) angeordnet ist, wobei die Probenkammeranordnung an einem Teil ihrer äußeren Oberfläche eine im Inneren des Trägerrohres (1) angeordnete Kupplungsvorrichtung (14) zur Ankopplung einer Trägerlanze zum Eintauchen des Trägerrohres aufweist, wobei die Probenkammeranordnung eine innere Wand für den Probenraum (9) und eine äußere Wand (12, 12') aufweist, wobei die äußere Wand (12, 12') die innere Wand mindestens teilweise beabstandet derart umgibt, dass zwischen äußerer Wand (12, 12') und innerer Wand ein Hohlraum (15) angeordnet ist.

2. Probennehmer nach Anspruch 1, **dadurch gekennzeichnet, dass** die Probenkammeranordnung mehrere, den Probenraum (9) unmittelbar umgebende und voneinander lösbare Teile aufweist, wobei mindestens eines der Teile im Inneren des Trägerrohres (1) angeordnet ist.

3. Probennehmer nach einem der Ansprüche 1 oder 2, **dadurch gekennzeichnet, dass** die Probenkammeranordnung oder der die Kupplungsvorrichtung (14) aufweisende Teil der Probenkammeranordnung am Eintauchende (21) des Trägerrohres (1) derart angeordnet ist, dass die Probenkammeranordnung oder der die Kupplungsvorrichtung (14) aufweisende Teil durch das Innere des Trägerrohres (1) hindurch zu dem dem Eintauchende (21) abgewandten Ende des Trägerrohres (1) und dort aus dem Trägerrohr (1) heraus bewegbar ist.

4. Probennehmer nach einem der Ansprüche 1 bis 3, **dadurch gekennzeichnet, dass** die Probenkammeranordnung oder der die Kupplungsvorrichtung (14) aufweisende Teil der Probenkammeranordnung einen Querschnitt senkrecht zur Längsachse des Trägerrohres (1) aufweist, der höchstens so groß ist wie der Querschnitt des Inneren des Trägerrohres (1) senkrecht zu seiner Längsachse.

5. Probennehmer nach Anspruch 2, **dadurch gekennzeichnet, dass** die Probenkammeranordnung ein erstes Teil und ein zweites Teil aufweist, die gemeinsam den Probenraum (9) umgeben, dass das Trägerrohr (1) ein die Kupplungsvorrichtung (14) enthaltendes Hauptteil und ein am Eintauchende (21) des Trägerrohres (1) vom Hauptteil lösbar angeordnetes Endteil aufweist, wobei das erste Teil der Probenkammeranordnung an dem Hauptteil und das zweite Teil der Probenkammeranordnung an dem Endteil des Trägerrohres (1) fixiert ist.

6. Probennehmer nach Anspruch 5, **dadurch gekennzeichnet, dass** das Endteil mittels einer Steck- oder Schraubverbindung mit dem Hauptteil verbunden ist.

7. Probennehmer nach Anspruch 5 oder 6, **dadurch gekennzeichnet, dass** das Endteil mittels Klammern oder Krampen mit dem Hauptteil verbunden ist.

8. Probennehmer nach einem der Ansprüche 1 bis 7, **dadurch gekennzeichnet, dass** die Kupplungsvorrichtung (14) als Rastkupplung oder als Bajonettkupplung oder als Schraubkupplung ausgebildet ist.

9. Probennehmer nach einem der Ansprüche 1 bis 8, **dadurch gekennzeichnet, dass** das Verhältnis der Masse der in dem Probenraum (9) aufgenommenen Schmelze zur Masse der Probenkammeranordnung ohne Schmelze kleiner als 0,8, vorzugsweise höchstens 0,1 ist.

10. Probennehmer nach einem der Ansprüche 1 bis 9, **dadurch gekennzeichnet, dass** die Kupplungsvorrichtung (14) mindestens einen Gasdurchlaufkanal aufweist, der durch die äußere Wand (12, 12') der Probenkammeranordnung hindurch oder an diese heran führt.

11. Probennehmer nach einem der Ansprüche 1 bis 10, mit der Probenkammeranordnung mit dem Probenraum (9), der von einer aus mehreren Teilen gebildeten inneren Wand unmittelbar umgeben ist und einem mit dem Probenraum (9) verbundenen Einlaufrohr (2) zur Aufnahme einer Probe (20) aus einer Metall- oder Kryolithschmelze, insbesondere einer Stahlschmelze in dem Probenraum (9), wobei das Einlaufrohr (2) mit einer Einauföffnung in den Probenraum (9) mündet, **dadurch gekennzeichnet, dass** das Verhältnis V zwischen Masse M der Probe (20) und dem Material der inneren Wand durch folgendes Verhältnis charakterisiert ist

$$V = \frac{M \times 24000}{m \times c \times \lambda} < 0{,}15 \;,$$ wobei m die Masse der inneren Wand, c die spezifische Wärmekapazität und λ die thermische Leitfähigkeit des Materials der inneren Wand sind.

**12.** Probennehmer nach Anspruch 11, **dadurch gekennzeichnet, dass** V < 0,05 ist.

**13.** Probennehmer nach Anspruch 11 oder 12, **dadurch gekennzeichnet, dass** das Verhältnis von Volumen des Probenraumes (9) zum Gesamtquerschnitt der der Entlüftung dienenden Öffnungen ist kleiner als 500 mm, vorzugsweise kleiner als 100 mm.

**14.** Probennehmer nach einem der Ansprüche 11 bis 13, **dadurch gekennzeichnet, dass** sie mindestens zwei den Probenraum (9) unmittelbar umgebende und voneinander lösbare Teile aufweist.

**15.** Probennehmer nach einem der Ansprüche 11 bis 14, **dadurch gekennzeichnet, dass** das Einlaufrohr (2) an der Einlauföffnung einen verringerten Querschnitt aufweist.

**16.** Verfahren zur Probennahme aus Schmelzen mit einer Schmelztemperatur von größer 600 °C, insbesondere für Metall- oder Kryolithschmelzen, mit einem Probennehmer nach einem der Ansprüche 1 bis 10, wobei durch das einem Eintauchende (21) des Trägerrohres (1) entgegengesetzten Ende hindurch eine Trägerlanze in das Trägerrohr (1) eingeschoben wird, wobei die Trägerlanze an die Kupplungsvorrichtung (14) der Probenkammeranordnung angekoppelt wird, wobei danach das Eintauchende (21) des Trägerrohres (1) in die Schmelze eingetaucht und der Probenraum (9) der Probenkammeranordnung mit Schmelze gefüllt wird, wobei anschließend die Probenkammeranordnung oder der die Kupplungsvorrichtung (14) aufweisende Teil der Probenkammeranordnung mit der Trägerlanze durch das Trägerrohr (1) hindurch aus dem dem Eintauchende (21) entgegengesetzten Ende des Trägerrohres (1) herausgezogen wird, wobei nach dem Herausziehen des die Kupplungsvorrichtung (14) aufweisenden Teils der Probenkammeranordnung aus dem Trägerrohr (1), wobei ein Teil der Oberfläche der in der Probenkammeranordnung befindlichen Probe (20) in unmittelbaren Kontakt mit der Umgebung der Probenkammeranordnung tritt, eine ein Spektrometer (16) aufweisende Lanze in das Trägerrohr (1) eingeschoben und die Oberfläche der Probe (20) mit Hilfe des Spektrometers (16) analysiert wird.

**17.** Verfahren zur Probennahme aus Schmelzen mit einer Schmelztemperatur von größer 600 °C, insbesondere für Metall- oder Kryolithschmelzen, mit einem Probennehmer nach einem der Ansprüche 1 bis 10, wobei durch das einem Eintauchende (21) des Trägerrohres (1) entgegengesetzten Ende hindurch eine Trägerlanze in das Trägerrohr (1) eingeschoben wird, wobei die Trägerlanze an die Kupplungsvorrichtung (14) der Probenkammeranordnung angekoppelt wird, wobei danach das Eintauchende (21) des Trägerrohres (1) in die Schmelze eingetaucht und der Probenraum (9) der Probenkammeranordnung mit Schmelze gefüllt wird, wobei anschließend die Probenkammeranordnung oder der die Kupplungsvorrichtung (14) aufweisende Teil der Probenkammeranordnung mit der Trägerlanze durch das Trägerrohr (1) hindurch aus dem dem Eintauchende (21) entgegengesetzten Ende des Trägerrohres (1) herausgezogen wird, wobei nach dem Herausziehen des die Kupplungsvorrichtung (14) aufweisenden Teils der Probenkammeranordnung aus dem Trägerrohr (1), wobei ein Teil der Oberfläche der in der Probenkammeranordnung befindlichen Probe (20) in unmittelbaren Kontakt mit der Umgebung der Probenkammeranordnung tritt, eine einen Greifer (19) aufweisende Lanze in das Trägerrohr (1) eingeschoben wird, der Greifer (19) die Probe (20) ergreift, diese aus der Probenkammeranordnung entnimmt und durch das Trägerrohr (1) hindurch aus dem dem Eintauchende (21) entgegengesetzten Ende des Trägerrohres (1) herauszieht.

**18.** Verfahren nach Anspruch 17, **dadurch gekennzeichnet, dass** die Proben nach dem Herausziehen aus dem Trägerrohr (1) einer Analyseneinrichtung zugeführt wird.

**19.** Verfahren nach mindestens einem der Ansprüche 16 bis 18, **dadurch gekennzeichnet, dass** während und/oder nach der Probennahme an die Probenkammeranordnung oder in einen Hohlraum (15) der Probenkammeranordnung ein Inertgas geleitet wird.

**Claims**

**1.** A sampler for taking samples from melts having a melting point higher than 600 °C, more particularly for metal or cryolite melts, the sampler comprising a carrier pipe (1) having an immersion end (21) and a sample chamber arrangement arranged at the immersion end (21) of the carrier pipe (1) and having an inlet opening and a sample chamber (9) for the melt, the sample chamber arrangement being arranged in the interior region of the carrier pipe (1) at least in certain regions, wherein the sample chamber arrangement has at a portion of its outer surface a coupling device (14) for coupling a carrier lance for immersing the carrier pipe, the coupling device (14) being arranged in the interior region of the carrier pipe (1), wherein the sample chamber arrangement has an inner wall for the sample chamber (9) and an outer wall (12, 12'), wherein the outer wall (12, 12') surrounds the inner wall and is at least in part spaced apart therefrom such that a cavity is arranged between the outer wall (12, 12') and the inner wall.

2. The sampler in accordance with Claim 1, **characterised in that** the sample chamber arrangement has a plurality of parts that directly surround the sample chamber (9) and are detachable from each other, wherein at least one of said parts is arranged in the interior region of the carrier pipe (1).

3. The sampler in accordance with any one of Claims 1 or 2, **characterised in that** the sample chamber arrangement or the part of the sample chamber arrangement having the coupling device (14) is arranged at the immersion end (21) of the carrier pipe (1) such that the sample chamber arrangement or the part having the coupling device (14) can be moved through the interior region of the carrier pipe (1) to the end of the carrier pipe (1) facing away from the immersion end (21) and, from there, out of the carrier pipe (1).

4. The sampler in accordance with any one of Claims 1 to 3, **characterised in that** the sample chamber arrangement or the part of the sample chamber arrangement having the coupling device (14) has a cross-section extending perpendicularly to the longitudinal axis of the carrier pipe (1), said cross-section being no larger than the cross-section of the interior region of the carrier pipe (1) extending perpendicularly to its longitudinal axis.

5. The sampler in accordance with Claim 2, **characterised in that** the sample chamber arrangement has a first part and a second part which jointly surround the sample chamber (9), that the carrier pipe (1) has a main part containing the coupling device (14) and an end part that is arranged at the immersion end (21) of the carrier pipe (1) such that it can be detached from the main part, wherein the first part of the sample chamber arrangement is fixed to the main part and the second part of the sample chamber arrangement is fixed to the end part of the carrier pipe (1).

6. The sampler in accordance with Claim 5, **characterised in that** the end part is connected to the main part by means of a plugged or screwed connection.

7. The sampler in accordance with Claim 5 or 6, **characterised in that** the end part is connected to the main part by means of clamps or cramps.

8. The sampler in accordance with any one of Claims 1 to 7, **characterised in that** the coupling device (14) is formed as a catch coupling or a bayonet coupling or a screwed coupling.

9. The sampler in accordance with any one of Claims 1 to 8, **characterised in that** the ratio of the mass of the melt received in the sample chamber (9) to the mass of the sample chamber arrangement without the melt is less than 0.8, preferably no more than 0.1.

10. The sampler in accordance with any one of Claims 1 to 9, **characterised in that** the coupling device (14) has at least one gas flow duct that passes through or approaches the outer wall (12, 12') of the sample chamber arrangement.

11. The sampler in accordance with any one of Claims 1 to 10, comprising the sample chamber arrangement having the sample chamber (9) which is directly surrounded by an inner wall formed from a plurality of parts and comprising an inlet pipe (2) that is connected to the sample chamber (9) and receives a sample from a metal or cryolite melt, more particularly a steel melt in the sample chamber (9), wherein the inlet pipe (2) ends in the sample chamber (9) with an inlet opening, **characterised in that** the ratio V of the mass M of the sample (20) to the material of the inner wall is **characterised by** the following ratio:

$$V = \frac{M \times 24000}{m \times c \times \lambda} < 0.15,$$

wherein m is the mass of the inner wall, c is the specific heat capacity and λ is the thermal conductivity of the material of the inner wall.

12. The sampler in accordance with Claim 11, **characterised in that** V is < 0.05.

13. The sampler in accordance with Claim 11 or 12, **characterised in that** the ratio of the volume of the sample chamber (9) to the total cross-section of the openings serving for ventilation is less than 500 mm, preferably less than 100 mm.

14. The sampler in accordance with any one of Claims 11 to 13, **characterised in that** it has two parts that directly surround the sample chamber (9) and are detachable from each other.

15. The sampler in accordance with any one of Claims 11 to 14, **characterised in that** the inlet pipe (2) has a reduced cross-section at the inlet opening.

16. A method for taking samples from melts having a melting temperature of more than 600 °C, more particularly for metal or cryolite melts, using a sampler in accordance with any one of Claims 1 to 10, wherein a carrier lance is pushed through the end opposite to an immersion end (21) of the carrier pipe (1) and into the carrier pipe (1), wherein the carrier lance is coupled to the coupling device (14) of the sample chamber arrangement, wherein thereafter the immersion end (21) of the carrier pipe (1) is immersed into the melt and the sample chamber (9) of the sample chamber arrangement is filled with melt, wherein subsequently the sample chamber arrangement or the part of the sample chamber arrangement having the coupling device (14) is pulled through the carrier pipe (1) and out of the end of the carrier pipe (1) opposite to the immersion end (21) using the carrier lance, wherein, after the part of the sample chamber arrangement having the coupling device (14) has been pulled out of the carrier pipe (1) with a portion of the surface of the sample (20) present in the sample chamber arrangement coming into direct contact with the environment of the sample chamber arrangement, a lance having a radiation spectrometer (16) is pushed into the carrier pipe (1) and the surface of the sample (20) is analysed using the radiation spectrometer (16).

17. The method for taking samples from melts having a melting temperature of more than 600 °C, more particularly for metal or cryolite melts, using a sampler in accordance with any one of Claims 1 to 10, wherein a carrier lance is pushed through the end opposite to an immersion end (21) of the carrier pipe (1) and into the carrier pipe (1), wherein the carrier lance is coupled to the coupling device (14) of the sample chamber arrangement, wherein thereafter the immersion end (21) of the carrier pipe (1) is immersed into the melt and the sample chamber (9) of the sample chamber arrangement is filled with melt, wherein subsequently the sample chamber arrangement or the part of the sample chamber arrangement having the coupling device (14) is pulled through the carrier pipe (1) and out of the end of the carrier pipe (1) opposite to the immersion end (21) using the carrier lance, wherein, after the part of the sample chamber arrangement having the coupling device (14) has been pulled out of the carrier pipe (1) with a portion of the surface of the sample (20) present in the sample chamber arrangement coming into direct contact with the environment of the sample chamber arrangement, a lance having a gripper (19) is pushed into the carrier pipe (1), the gripper (19) seizes the sample (20), takes the same out of the sample chamber arrangement and pulls the same through the carrier pipe (1) and out of the end of the carrier pipe (1) opposite to the immersion end (21).

18. The method in accordance with Claim 17, **characterised in that** the sample, after having been pulled out of the carrier pipe (1), is delivered to an analysing device.

19. The method in accordance with at least any one of Claims 16 to 18, **characterised in that**, while the sample is being taken and/or after the sample has been taken, an inert gas is introduced to the sample chamber arrangement or into a cavity (15) of the sample chamber arrangement.

**Revendications**

1. Echantillonneur pour l'échantillonnage de matières fondues avec un point de fusion supérieur à 600 °C, notamment pour les masses fondues de métal ou de cryolithe avec un tube support (1) présentant une extrémité d'immersion (21) et une disposition de chambre d'échantillon disposée à l'extrémité d'immersion (21) du tube support (1), présentant une ouverture d'entrée et un espace échantillon (9) pour les matières fondues, laquelle est disposée au moins partiellement à l'intérieur du tube support (1), la disposition de chambre d'échantillon présentant, sur une partie de sa surface extérieure, un dispositif de couplage (14) disposé à l'intérieur du tube support (1) pour le couplage d'une lance support pour l'immersion du tube support,
la disposition de chambre d'échantillon présentant une paroi intérieure pour l'espace échantillon (9) et une paroi extérieure (12, 12'), la paroi extérieure (12, 12') entourant la paroi intérieure en étant au moins partiellement espacée de telle manière à ce qu'une cavité (15) soit placée entre la paroi extérieure (12, 12') et la paroi intérieure.

2. Echantillonneur conformément à la revendication n°1, **caractérisé en ce que** la disposition de chambre d'échantillon présente plusieurs parties entourant directement l'espace échantillon (9) et détachables les unes des autres, au moins l'une des parties étant disposées à l'intérieur du tube support (1).

3. Echantillonneur conformément à l'une des revendications n°1 ou n°2, **caractérisé en ce que** la disposition de chambre d'échantillon ou la partie de la disposition de chambre d'échantillon à l'extrémité d'immersion (21) du tube support (1), présentant le dispositif de couplage (14), est disposée de telle manière à ce que la disposition de chambre d'échantillon ou la partie présentant le dispositif de couplage (14) passe par l'intérieur du tube support (1) vers l'extrémité du tube support (1), détournée de l'extrémité d'immersion (21), et soit enlevable là du tube support (1).

**4.** Echantillonneur conformément à l'une des revendications n°1 à n°3, **caractérisé en ce que** la disposition de chambre d'échantillon ou la partie de la disposition de chambre d'échantillon, présentant le dispositif de couplage (14), présente une coupe transversale perpendiculaire à l'axe longitudinal du tube support (1) qui est au maximum aussi grande que la coupe transversale de l'intérieur du tube support (1) perpendiculaire à son axe longitudinal.

**5.** Echantillonneur conformément à la revendication n°2, **caractérisé en ce que** la disposition de chambre d'échantillon présente une première partie et une deuxième partie qui entourent ensemble l'espace échantillon (9), que le tube support (1) présente une partie principale contenant le dispositif de couplage (14) et une partie terminale disposée de façon amovible de la partie principale, à l'extrémité d'immersion (21) du tube support (1), la première partie de la disposition de chambre d'échantillon étant fixée sur la partie principale et la deuxième partie de la disposition de chambre d'échantillon sur la partie terminale du tube support (1).

**6.** Echantillonneur conformément à la revendication n°5, **caractérisé en ce que** la partie terminale est reliée à la partie principale au moyen d'un raccord enfichable ou vissable.

**7.** Echantillonneur conformément à la revendication n°5 ou n°6, **caractérisé en ce que** la partie terminale est reliée à la partie principale au moyen d'agrafes ou de crampes.

**8.** Echantillonneur conformément à l'une des revendications n°1 à n°7, **caractérisé en ce que** le dispositif de couplage (14) est formé comme raccord à enclenchement ou comme raccord à baïonnette ou comme raccord à vis.

**9.** Echantillonneur conformément à l'une des revendications n°1 à n°8, **caractérisé en ce que** le rapport de la masse de la matière fondue contenue dans l'espace échantillon (9) par rapport à la masse de la disposition de chambre d'échantillon sans matière fondue est inférieur à 0,8, de préférence au plus de 0,1.

**10.** Echantillonneur conformément à l'une des revendications n°1 à n°9, **caractérisé en ce que** le dispositif de couplage (14) présente au moins un conduit de passage de gaz qui passe par la paroi extérieure (12, 12') de la disposition de chambre d'échantillon ou se rapproche de celle-ci.

**11.** Echantillonneur conformément à l'une des revendications n°1 à n°10, avec la disposition de chambre d'échantillon avec l'espace échantillon (9) qui est directement entouré d'une paroi intérieure formée de plusieurs parties et avec un tube d'entrée (2) relié à l'espace échantillon (9) pour la réception d'un échantillon (20) d'une masse fondue de métal ou de cryolithe, notamment une masse d'acier fondue dans l'espace échantillon (9), le tube d'entrée (2) avec une ouverture d'entrée conduisant dans l'espace échantillon (9), **caractérisé en ce que** le rapport V entre la masse M de l'échantillon (20) et la matière de la paroi intérieure est **caractérisé par** le rapport suivant :

$$V = \frac{M \times 24000}{m \times c \times \lambda} < 0,15$$

m étant la masse de la paroi intérieure, c la capacité thermique spécifique et λ la conductibilité thermique de la matière de la paroi intérieure.

**12.** Echantillonneur conformément à la revendication n°11, **caractérisé en ce que** V est < 0,05.

**13.** Echantillonneur conformément à la revendication n°11 ou n°12, **caractérisé en ce que** le rapport du volume de l'espace échantillon (9) par rapport à la coupe transversale totale des ouvertures servant à la ventilation est inférieur à 500 mm, de préférence inférieur à 100 mm.

**14.** Echantillonneur conformément à l'une des revendications n°11 à n°13, **caractérisé en ce qu'**il présente au moins deux parties entourant directement l'espace échantillon (9) et détachables l'une de l'autre.

**15.** Echantillonneur conformément à l'une des revendications n°11 à n°14, **caractérisé en ce que** le tube d'entrée (2) présente, à l'ouverture d'entrée, une coupe transversale diminuée.

**16.** Procédé pour l'échantillonnage de matières fondues avec une température de fusion supérieure à 600°C, notamment pour les masses fondues de métal ou de cryolithe, avec un échantillonneur conformément à l'une des revendications n°1 à n°10, une lance support étant insérée dans le tube support (1) par l'extrémité opposée à une extrémité d'immersion (21) du tube support (1), la lance support étant couplée au dispositif de couplage (14) de la disposition de chambre d'échantillon, après quoi l'extrémité d'immersion (21) du tube support (1) est immergée dans la matière fondue et l'espace échantillon (9) de la disposition de la chambre d'échantillon est rempli de matière fondue, la disposition de chambre d'échantillon ou la partie de la disposition de chambre d'échantillon, présentant le dispositif de couplage (14), étant retirée avec la lance support par le tube support (1) de l'extrémité du tube support (1), opposée à l'extrémité d'immersion (21),

après le retrait de la partie de la disposition de chambre d'échantillon, présentant le dispositif de couplage (14), du tube support (1), une partie de la surface de l'échantillon (20) se trouvant dans la disposition de chambre d'échantillon, entrant en contact direct avec l'environnement de la disposition de chambre d'échantillon, une lance présentant un spectromètre (16) étant insérée dans le tube support (1) et la surface de l'échantillon (20) étant analysée à l'aide du spectromètre (16).

17. Procédé pour l'échantillonnage de matières fondues avec une température de fusion supérieure à 600°C, notamment pour les masses fondues de métal ou de cryolithe, avec un échantillonneur conformément à l'une des revendications n°1 à n°10, une lance support étant insérée dans le tube support (1) par l'extrémité opposée à une extrémité d'immersion (21) du tube support (1), la lance support étant couplée au dispositif de couplage (14) de la disposition de chambre d'échantillon, après quoi l'extrémité d'immersion (21) du tube support (1) est immergée dans la matière fondue et l'espace échantillon (9) de la disposition de chambre d'échantillon est rempli de matière fondue, la disposition de chambre d'échantillon ou la partie de la disposition de chambre d'échantillon, présentant le dispositif de couplage (14), étant retirée avec la lance support par le tube support (1) de l'extrémité du tube support (1), opposée à l'extrémité d'immersion (21), après le retrait de la partie de la disposition de chambre d'échantillon, présentant le dispositif de couplage (14), du tube support (1), une partie de la surface de l'échantillon (20) se trouvant dans la disposition de chambre d'échantillon, entrant en contact direct avec l'environnement de la disposition de chambre d'échantillon, une lance présentant un grappin (19) étant insérée dans le tube support (1), le grappin (19) saisissant l'échantillon (20), enlevant ce dernier de la disposition de chambre d'échantillon et le retirant, par le tube support (1), de l'extrémité du tube support (1), opposée à l'extrémité d'immersion (21).

18. Procédé conformément à la revendication n°17, **caractérisé en ce que** l'échantillon est amené à un dispositif d'analyse après avoir été retiré du tube support (1).

19. Procédé conformément au moins à l'une des revendications n°16 à n°18, **caractérisé en ce qu'**un gaz inerte est acheminé vers la disposition de chambre d'échantillon ou dans une cavité (15) de la disposition de chambre d'échantillon pendant et/ou après l'échantillonnage.

**Fig. 1**

Fig. 2

**Fig. 3**

Fig. 4

**Fig. 5**

**Fig. 6**

**Fig. 7**

**Fig. 8**

**Fig. 9**

10

30

32

31

32

10

Fig. 10

Fig. 11

36    11
      12
       9
10
      13
7
23    12'

25
      2

24

**Fig. 12**

36

12

11

10

7

12'

23

2

25

24

**Fig. 13**

**EP 2 525 207 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 3233677 C1
- EP 1544591 A2
- DE 2504583 A1
- DE 102005060492 B3
- DE 4303687 C1
- EP 143498 A2
- US 4893516 A

27